# EUROPÄISCHE PATENTSCHRIFT

(11) **EP 0 657 177 B1**
(45) Veröffentlichungstag und Bekanntmachung des Hinweises auf die Patenterteilung: **21.04.1999**
(21) Anmeldenummer: 94118915.1
(22) Anmeldetag: 01.12.1994
(51) Int. Cl.: A61L 2/26, C12Q 1/22

(54) **Vorrichtung zum Testen und Kontrollieren von nach dem Vakuumverfahren arbeitenden Dampfsterilisatoren**
Device for testing and controlling of steam sterilizers working according to vacuum process
Dispositif pour tester et contrôler des sterilisateurs à vapeur fonctionnant selon le procédé sous vide

(30) Priorität: 08.12.1993 DE 9318855 U; 18.09.1994 DE 9415097 U
(43) Veröffentlichungstag der Anmeldung: 14.06.1995
(73) Patentinhaber: SECUNDUS Medizinische Kontrollsysteme GmbH, 41516 Grevenbroich (DE)
(72) Erfinder: Brink, Maria, D-47918 Tönisvorst (DE); Schefter, Siegfried, 41516 Grevenbroich (DE)
(74) Vertreter: von Creytz, Dietrich, Dipl.-Phys.

(56) Entgegenhaltungen:
- EP-A- 0 419 282
- DE-U- 9 401 058
- DE-U- 9 415 097

## Beschreibung

Die Erfindung betrifft eine Vorrichtung zum Testen und Kontrollieren von nach dem Vakuumverfahren arbeitenden Dampfsterilisatoren, mit einem einen Boden und Seitenwände aufweisenden Behältnis aus gas- und flüssigkeitsundurchlässigem Werkstoff zur Aufnahme eines Indikators in Nähe des Bodeninneren und eines darüber anzuordnenden, fasrigen Füllstoffes.

Eine derartige Vorrichtung ist aus der EP 0 419 282 B1 bekannt. Diese bekannte Vorrichtung ist als Einweg-Vorrichtung zur einmaligen Benutzung ausgebildet. Das Behältnis dieser Vorrichtung ist an beiden Stirnenden verschlossen, wobei in einem Stirnende Öffnungen vorgesehen sind. Der Indikator ist im Inneren des Behältnisses in Nähe des keine Öffnung aufweisenden, geschlossenen Stirnendes angeordnet. Zur Durchführung des Test- und Kontrollierprozesses wird das Behältnis in die Kammer eines Dampfsterilisators eingebracht, welche anschließend evakuiert wird. Dann wird Dampf in die Kammer eingelassen. Um den in Nähe des geschlossenen Stirnendes angeordneten Indikator beeinflussen und ggf. einen Farbumschlag herbeiführen zu können, muß der durch die Öffnungen in dem gegenüberliegenden Stirnende in das Innere des Behältnisses eintretende Dampf den gesamten porösen, fasrigen Füllstoff durchdringen. Das geschlossene Stirnende ist nach Art eines Abreißdeckels ausgebildet, der nach Durchführung des Test- und Kontrolliervorganges zwecks Herausnehmens und Prüfens des Indikators entfernt wird. Infolge des Aufreißvorganges ist diese Vorrichtung nach einmaligem Gebrauch nicht wiederverwendbar. Der abreißbare Deckel ist über eine als Sollbruchstelle wirkende Schwächungslinie angebracht. Da bei dem zum Testen und Kontrollieren durchzuführenden Sterilisationsprozeß infolge des zwischen den Öffnungen und dem Indikator vorgesehenen Füllstoffes erhebliche Druckdifferenzen zwischen dem den Indikator aufweisenden Bereich und dem Breich außerhalb des Behältnisses auftreten, können sich im Bereich der zur Anbringung des abreißbaren Deckels erforderlichen Schwächungslinien während des Prozesses Risse, Öffnungen od. dgl. bilden, durch deren Vorhandensein das Testergebnis völlig verfälscht werden kann, da in einem solchen Fall ein direkter Dampfkontakt des Indikators ohne vorherige Dampfdurchdringung des Füllstoffes auftritt. Eine Feststellung, ob sich während des Prozesses Haarrisse, Öffnungen od. dgl. gebildet haben, ist nur sehr schwer bzw. im praktischen Betrieb überhaupt nicht möglich, da zur Betrachtung des Indikators der Abreißdeckel entfernt wird und dann natürlich nicht mehr erkennbar ist, ob der Abreißdeckel während des gesamten Prozesses dicht schließend angebracht war.

Ein weiterer Nachteil bei dieser bekannten Vorrichtung ist der direkte Kontakt des Indikators mit dem geschlossenen Stirnende des Behältnisses. Ziel bei der Verwendung der Vorrichtung soll sein, nicht kondensierbare Gase aufzuspüren, die die Sterilisation gefährden. Dazu muß sichergestellt sein, daß die Gase, wie z.B. Luft, während der Einwirkzeit des Dampfes im Indikatorbereich verharrt und den Kontakt des Dampfes mit dem chemischen Farbindikator verhindert. Luft hat bei ansteigender Temperatur das Bestreben dorthin zu strömen und zu verharren, wo die Temperatur niedriger ist als die Umgebungstemperatur. Nimmt die Luft während der Sterilisierphase fast zeitgleich die Temperatur der Wandung und des geschlossenen Stirnendes des Behältnisses sowie des porösen, fasrigen Füllstoffes an, beginnt sie zu strömen, verläßt den Indikatorbereich und treibt durch den porösen, fasrigen Füllstoff den Behältnisöffnungen entgegen, da dort durch das ständige Kondensieren des Dampfes ein Unterdruck herrscht. Folglich kann der Dampf ungehindert an den chemischen Farbindikator gelangen und durch dessen Farbumschlag ein falsches Test- und Kontrollergebnis verursachen.

Zur Behebung der geschilderten Nachteile liegt der Erfindung dir Aufgabe zugrunde, eine Vorrichtung der eingangs genannten Art so zu gestalten, daß infolge der bei dem Sterilisationsprozeß auftretenden Druckdifferenzen und thermophysikalischen Einflüssen keine Verfälschung des Test- und Kontrollergebnisses erfolgen kann. Eine weitere Aufgabe der Erfindung besteht darin, die Vorrichtung so umzugestalten, daß sie nicht nur einmal, sondern wesentlich öfter verwendbar ist. Eine weitere Aufgabe der Erfindung besteht darin, im Vergleich zu anderen bekannten Vorrichtungen zum Testen und Kontrollieren von nach dem Vakuumverfahren arbeitenden Dampfsterilisatoren mit wesentlich weniger Verbrauchsmaterialien pro Vorgang auszukommen, wobei auch die Möglichkeit bestehen soll, das Verbrauchsmaterial, nämlich den porösen, fasrigen Füllstoff, mehrfach zu verwenden.

Erfindungsgemäß ist vorgesehen, daß das Behältnis einstückig mit durchgehender Wandung des Bodens und der Seitenwände ohne Schwächungsbereiche und Schwächungsstellen ausgebildet ist, so daß infolge der bei dem Sterilisationsprozeß auftretenden Druckdifferenzen zwischen dem dem Boden zugewandten Innenraum des Behältnisses und dem Behältnisäußeren keine Luft- und Dampfdurchtrittsöffnungen in der Wandung entstehen können, und daß das Behältnis auf der dem Boden gegenüberliegenden Seite offen ausgebildet ist.

Infolge der ohne Schwächungsbereiche, Schwächungsstellen od. dgl. ausgebildeten durchgehenden Wandung des Bodens und der Seitenwände des Behältnisses können die bei der Vorrichtung nach dem Stand der Technik infolge der dort vorgesehenen Schwächungsbereiche möglichen Luft- und Dampfdurchtrittsöffnungen nicht auftreten, so daß keine Verfälschung des Testergebnisses infolge der während des Prozesses naturgemäß auftretenden Druckdifferenzen eintreten kann. Dadurch, daß das Behältnis auf der dem Boden gegenüberliegenden Seite offen ausgebildet ist, kann zum Herausnehmen des Indikators nach Durchführung des Prozesses in einfacher Weise der poröse, fasrige Füllstoff herausgenommen bzw. entfernt werden, so dann der Indikator ohne weiteres zugänglich ist. Das Behältnis bleibt stets unverändert, da kein Deckel od. dgl. entfernt werden muß. Das Behältnis kann daher grundsätzlich beliebig oft verwendet werden, während der poröse, fasrige Füllstoff mehrfach verwendet werden kann. Die Anzahl der Verwendungsmöglichkeiten des Füllstoffes ist von verschiedenen Parametern abhängig.

Erfindungsgemäß ist weiters vorgesehen, den Innenboden des Behältnisses mit einer Wärmeisolierung zu versehen. Dies kann so ausgeführt sein, daß das Bodeninnere des Behältnisses mit einem Flächengebilde aus temperatur-und feuchtigkeitsbeständigem Werkstoff ganzflächig bedeckt ist, und daß der Werkstoff des Flächengebildes eine wesentlich geringere Wärmeleitfähigkeit besitzt als der Werkstoff des Behältnisses. Das wärmeisolierende Flächengebilde soll dazu führen, daß sich Luft, die
a) durch nicht ausreichende Austreibung aus dem Inneren des Behältnisses in der Vakuumphase im Behältnisinneren zurückbleibt und/oder
b) durch eine Leckage in die Sterilisierkammer eindringt und/oder
c) mit dem Dampf als Inertgas in die Sterilisierkammer gelangt,
   nahe dem Innenboden des Behältnisses unmittelbar über dem wärmeisolierenden Flächengebilde einnistet und einen Kontakt des Dampfes mit dem chemischen Farbindikator verhindert; denn wo Luft ist, kann Dampf seine sterilisierende Wirkung nicht ausüben. Luft hat bei ansteigender Temperatur das Bestreben, dorthin zu strömen und zu verharren, wo die Temperatur niedriger ist als die Umgebungstemperatur. Da bei stetig ansteigender Temperatur und letztlich erreichter Sterilisiertemperatur von 121 bzw. 134 Grad C das Behältnis und der poröse, fasrige Füllstoff zeitgleich die Kammertemperatur annehmen, das wärmeisolierende Flächengebilde wegen der geringeren Wärmeleitfähigkeit länger kälter bleibt, verbleibt die schädliche Luft über dem wärmeisolierenden Flägebilde im Bereich des chemischen Farbindikators und verhindert den Kontakt des Dampfes mit dem chemischen Farbindikator und damit dessen Farbumschlag. Damit wird der Fehler im Prozeßverlauf angezeigt. In zweckmäßiger Ausgestaltung der Erfindung kann vorgesehen sein, daß der wärmeisolierende Werkstoff aus Silikon besteht. Die erfindungsgemäße Vorrichtung ist mit geringen Kosten herstellbar, arbeitet sehr umweltfreundlich, ist sehr handlich und kann mit so geringen Außenabmessungen hergestellt werden, daß die Prüfung sog. Kleinststerilisatoren in Arztpraxen möglich ist. Die Menge des in dem Behältnis verwendeten porösen, fasrigen Füllstoffes ist relativ klein, verglichen mit herkömmlichen ohne Behältnis verwendeten Testmaterialstapeln.

In zweckmäßiger Ausgestaltung der Erfindung kann vorgesehen sein, daß der poröse, fasrige Füllstoff aus hydrophilem Material besteht. Dadurch kann angezeigt werden, ob das Sterilisiermittel, nämlich der Wasserdampf, den vorgeschriebenen Sättigungsgrad besitzt.

In weiterer Ausbildung der Erfindung kann vorgesehen sein, daß der Füllstoff aus übereinandergeschichteten Lagen von Flächengebilden besteht. Diese Lagen können - etwa vergleichbar mit einer Rolle von zusammengepackten Münzen - in geeigneter Weise zusammengehalten sein. Wenn der poröse, fasrige Füllstoff aus übereinandergeschichteten Lagen von Flächengebilden gebildet ist, erhält man hierdurch einen Stapel, der in der Richtung von dem in der Nähe des Bodens angeordneten Indikator bis zum gegenüberliegenden offenen Stirnende eine ausreichende Konsistenz aufweist, um die zur Prüfung der Leistungsfähigkeit der Vakuumeinrichtung notwendige "Hindernishöhe" und Hinderniskonsistenz zu bilden.

Die Erfindung wird nachfolgend anhand des in der Zeichnung schematisch dargestellten Ausführungsbeispiels und eines Temperatur/Zeit-Diagramms näher erläutert. Es zeigen:
- Fig. 1: eine Seitenansicht eines Behältnisses;
- Fig. 2: eine Draufsicht auf das Behältnis;
- Fig. 3: eine Ansicht des Behältnisses von unten;
- Fig. 4: eine perspektivische Darstellung des Behältnisses mit einer Explosionsdarstellung der zur Durchführung des Test- und Kontrolliervorganges von nach dem Vakuumverfahren arbeitenden Dampfsterilisatoren in das Behältnis einzugebenden Inhaltsstoffe;
- Fig. 5: eine perspektivische Außenansicht des gefüllten Behältnisses.
- Fig. 6: Temperatur/Zeit-Diagramm über die Temperaturentwicklung in der Strömungsleitung einer Sterilisierkammer, je eines Behältnisses mit und ohne Wärmeisolierung und an der Außenwandung eines Behältnisses.

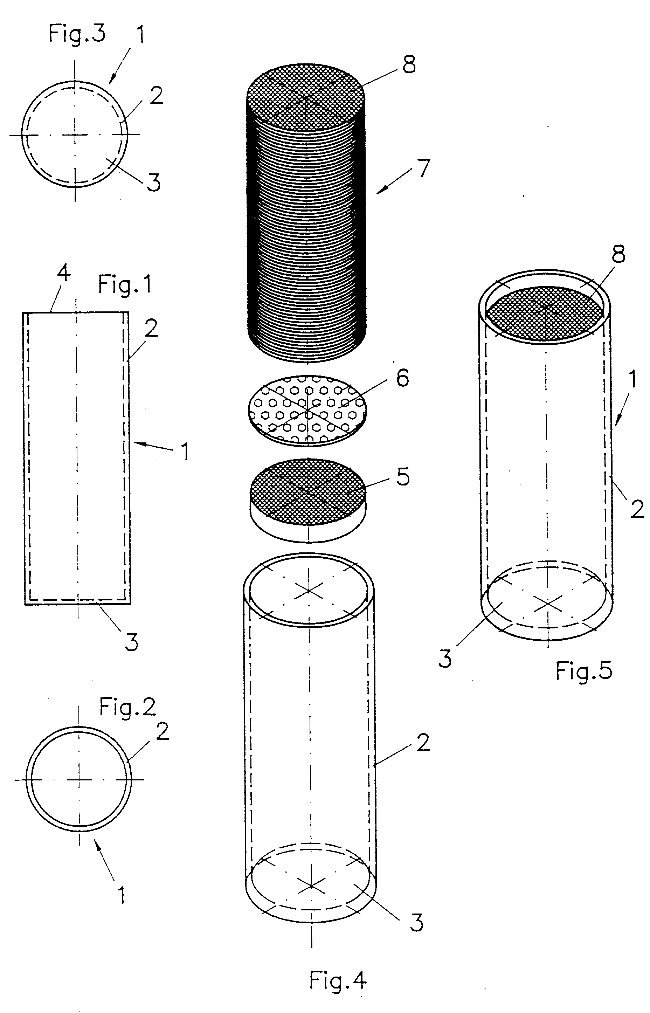

Das in den Figuren 1 bis 4 dargestellte Behältnis ist rohrförmig ausgebildet und weist einen Kreisquerschnitt auf. Das insgesamt mit 1 bezeichnete Behältnis hat Wände 2 und einen einstückig mit den Wänden 2 ausgebildeten Boden 3. Sowohl die Wände 2 und der Boden 3 als auch die Verbindungsbereiche zwischen Wänden und Boden sind mit durchgehender Wandung ohne Schwächungsbereiche und Schwächungsstellen ausgebildet. Auf der dem Boden 3 gegenüberliegenden Seite 4 ist das Behältnis offen ausgebildet.

Zum Testen und Kontrollieren von nach dem Vakuumverfahren arbeitenden Dampfsterilisatoren wird in das Behältnis 1 zunächst ein dem Innenprofil des Behältnisses entsprechend ausgebildetes Flächengebilde 5 aus temperatur- und feuchtigkeitsbeständigem Werkstoff mit geringerer Wärmeleitfähigkeit als die des Werkstoffs des Behältnisses 1 eingelegt, so daß der Innenboden des Behältnisses hiervon vollends bedeckt ist. Der chemische Farbindikator 6 wird auf die Oberseite des Flächengebildes 5 aufgelegt, und dann wird darüber ein insgesamt mit 7 bezeichneter poröser, fasriger Füllstoff aufgebracht. Der poröse, fasrige Füllstoff besteht bei dem dargestellten Ausführungsbeispiel aus übereinandergeschichteten Lagen von Flächengebilden. Die Lagen können einzeln aufgebracht werden; es besteht aber auch die Möglichkeit, die Lagen in geeigneter Weise zu einem Stapel zusammenzufassen. Fig. 5 zeigt die Vorrichtung mit gefülltem Behältnis, wobei die Oberseite 8 des porösen, fasrigen Füllstoffes sichtbar ist.

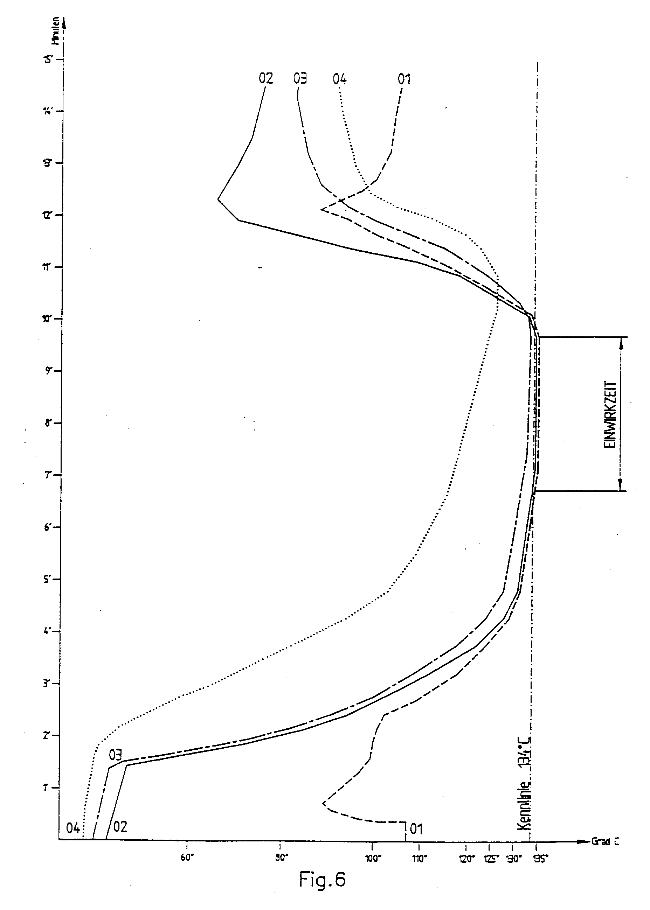

Figur 6 zeigt ein Diagramm über die Temperaturentwicklung an mehreren Meßpunkten in einer Sterilisierkammer in Korrelation mit der Zeit während eines Sterilisierprogramms mit verschlechtertem Vakuum; d.h., daß sich in den Behältnissen 1 erhebliche Mengen schädlicher Luft befinden. Die Temperaturen werden mit Thermoelementen gemessen. Die 4 Temperaturkurven werden wie folgt zugeordnet: #01 Strömungsleitung der Sterilisierkammer; #02 Außenwand des Behältnisses; #03 Behältnis 1 ohne wärmeisolierendes Flächengebilde 5; #04 Behältnis 1 mit wärmeisolierendem Flächengebilde 5.

Zum Testen und Kontrollieren eines nach dem Vakuumverfahren arbeitenden Dampfsterilisators wird eine Vorrichtung gemäß Fig. 5 in den Dampfsterilisator eingebracht. Bei diesem Vorgang wird zunächst eine Entlüftung herbeigeführt, und zwar dadurch, daß im Inneren des Dampfsterilisators mittels einer Vakuumpumpe ein Vakuum erzeugt wird. Da Vakuumpumpen üblicherweise nicht in der Lage sind, Luft aus porösen Stoffen und dementsprechend auch aus dem oberhalb des Indikators 6 angeordneten porösen Füllstoff in einem einzigen Arbeitsgang zu entfernen bzw. auszutreiben, geschieht dies in mehreren aufeinanderfolgenden Evakuierungsschritten, wobei zwischen den einzelnen Evakuierungsschritten Dampf in die Kammer des Sterilisators eingelassen wird, um dadurch das Erwärmen des zu entlüftenden porösen Gutes die Luftaustreibung zu erleichtern. Mittels der mehrmaligen Evakuierungsschritte wird erreicht, daß letztlich die Luft aus dem gesamten fasrigen Füllstoffstapel 7 bis auf einen geringen, unschädlichen Anteil von Restluft entfernt wird, wobei die in dem Füllstoffstapel enthaltene Luft jeweils nur durch den oberen Endbereich 8 austreten kann. Da der Stapel 7 das Innere des Behältnisses im wesentlichen ausfüllt, tritt der überaus größte Teil der in dem Stapel vor Testbeginn enthaltenen Luft durch die einzelnen Lagen des Stapels hindurch und nach oben aus. Der kritische Zustand des Stapels 7 ist abhängig von dem "Luftrückhaltewert" des verwendeten Materials. Je größer der Luftrückhaltewert ist, desto schwerer entlüftbar ist das Material. Je schwerer entlüftbar das Material ist, desto niedriger kann die Höhe des Füllstoffes in dem Behältnis sein.

Es erfolgt anschließend ein Dampfeinlaß in das Innere der Kammer. Um den Indikator beeinflussen zu können, muß dieser Dampf den Füllstoff 7 völlig durchdringen. Der Dampf muß den Füllstoff beginnend an dem oberen Stirnende 8 von oben nach unten durchdringen, um den Kontakt mit dem Indikator 6 zu bekommen. Der Indikator verändert bei Dampfkontakt in einer vorgegebenen Zeit seine Farbe, was zu einem Farbumschlag führt, der aussagt, ob der Dampfkontakt ausreichend war oder nicht. Gleichzeitig mit dem Einströmen des Dampfes erfolgt eine Druckerhöhung in der Kammer, um die übliche Sterilisiertemperatur von 121 bzw. 134 Grad C (Arbeitstemperatur) erreichen zu können. Um diese Temperatur zu halten, wird durch eine geeignete Steuerung bzw. Regelung ständig Dampf zugeführt, um sowohl die Temperatur als auch den Druck zu halten. Wenn eine bestimmte Einwirkzeit verstrichen ist, wird nach einer Trocknungsphase das Behältnis aus dem Dampfsterilisator herausgenommen. Dann entfernt man den Füllstoff 7, wodurch der Indikator 6 freigelegt wird. Das Aussehen des Indikators läßt erkennen, ob der Prozeß einwandfrei abgelaufen ist oder nicht.

Für einen weiteren Test kann dann das Behältnis 1 unter Verwendung des Füllstoffes 7, jedoch mit einem neuen Indikator 6, wiederverwendet werden.

## Patentansprüche

1. Vorrichtung zum Testen und Kontrollieren von nach dem Vakuumverfahren arbeitenden Dampfsterilisatoren, mit einem einen Boden (3) und Seitenwände (2) aufweisenden Behältnis aus gas- und flüssigkeitsundurchlässigem Werkstoff zur Aufnahme eines Indikators (6) in Nähe des Bodeninneren und eines darüber anzuordnenden porösen, fasrigen Füllstoffes (7), dadurch gekennzeichnet, daß das Behältnis (1) einstückig mit durchgehender Wandung des Bodens (3) und der Seitenwände (2) ohne Schwächungsbereiche und Schwächungsstellen ausgebildet ist, so daß infolge der bei dem Sterilisationsprozeß auftretenden Druckdifferenzen zwischen dem dem Boden (3) zugewandten Innenraum des Behältnisses (1) und dem Behältnisäußeren keine Luft- und Dampfdurchtrittsöffnunger in der Wandung entstehen können, und daß das Behältnis (1) auf der dem Boden (3) gegenüberliegenden Seite (4) offen ausgebildet ist.

2. Vorrichtung nach Anspruch 1, dadurch gekennzeichnet, daß das Bodeninnere (3) des Behältnisses (1) mit einem Flächengebilde (5) aus temperatur- und feuchtigkeitsbeständigem Werkstoff ganzflächig bedeckt ist, und daß der Werkstoff des Flächengebildes (5) eine wesentlich geringere Wärmeleitfähigkeit besitzt als der Werkstoff des Behältnisses (1).

3. Vorrichtung nach Anspruch 1, dadurch gekennzeichnet, daß der poröse, fasrige Füllstoff (7) aus hydrophilem Material besteht.

4. Vorrichtung nach Anspruch 1 oder 3, dadurch gekennzeichnet, daß der Füllstoff (7) aus übereinandergeschichteten Lagen von Flächengebilden besteht.

## Claims

1. Device for the testing and checking of steam sterilizers operating acc. to the vacuum treatment with a container made of a material which is non permeable for gas and liquid with bottom and side walls designed to accommodate an indicator (6) near the interior of the bottom and a porous fibrous filler (7) to be arranged above, The characteristics of which are that the container (1) is developed in one piece with seamless wall of the bottom (3) and side walls (2) without any weakening areas and weakening points. Therefore owing to the difference of pressure during the sterilizations process between the interior space of the container (1) facing the bottom (3) and the exterior of the container there is no chance of any air and steam gaps in the wall. The container (1) is open on the side opposing the bottom (3)

2. Device according. to claim 1 distinguished in such that the interior of the bottom (3) of the container (1) is fully covered with a surface formation (5) of temperature and moisture resistant material and that the material of the surface formation (5) has a considerable lower caloric conductibility than the material of the box (1).

3. Device acc. to claim 1, distinguished in such that the porous, fibrous filler (7) consists of hydrophilic material.

4. Device according. to claim 1 or 3, distinguished in such that the filler (7) consists of superimposed layers of surface formation.

## Revendications

1. Dispositif pour tester et contrôler selon le procédé à vide de stérilisateurs à vapeur avec réservoir doté de parois (2) et d'un fond (3) en matière imperméable aux gaz et aux liquides. Il est destiné à recevoir un indicateur (6) placé à l'intérieur du fond et dispose au-dessus par une matière de remplissage poreuse et fibreuse (7). Le réservoir (1) est formé d'une seule pièce, le fond (3) et les parois latérales (2), sans zone d'affaiblissement ou point faible, de sorte que la différence de pression par la procédure de stérilisation dirigée vers le fond (3) de l'espace intérieur et extérieur du réservoir (1) ne réprésente aucune ouverture dans la paroi. Par contre, grâce aux ouvertures placées du coté opposé (4) au fond (3) du réservoir (1), l'air et la vapeur peuvent s'échapper lors du processus de stérilisation.

2. Dispositif selon utilisation 1.Caractérisé par l'intérieur du fond (3) du réservoir (1) recouvert sur toute la surface d'une structure (5) en matière résistante à la température et à l'humidité. La matière de la structure de surface (5) possède une capacité de conduction de la chaleur nettement inférieure à la matière du réservoir.

3. Dispositif de l'utilisation 1. Caractérisé du fait que la matière de remplissage (7) poreuse et fibreuse est constituée d'une matière hydrophile.

4. Dispositif suivant 1. ou 3. Caractérisé par le fait que la matière de remplissage (7) est constituée de structures de surfaces en couches superposées.
